# EUROPÄISCHE PATENTSCHRIFT

(11) **EP 0 988 268 B1**
(45) Veröffentlichungstag und Bekanntmachung des Hinweises auf die Patenterteilung: **18.12.2002**
(21) Anmeldenummer: 99919162.0
(22) Anmeldetag: 30.03.1999
(51) Int. Cl.: C07C 37/82

(54) **VERFAHREN ZUM ENTFERNEN VON METALLIONEN AUS KRESOLGEMISCHEN**
METHOD FOR REMOVING METAL IONS FROM CRESOL MIXTURES
PROCEDE D'ELIMINATION D'IONS METAUX DANS DES MELANGES DE CRESOL

(30) Priorität: 01.04.1998 DE 19814555
(43) Veröffentlichungstag der Anmeldung: 29.03.2000
(73) Patentinhaber: RÜTGERS VFT AG, 44579 Castrop-Rauxel (DE)
(72) Erfinder: TALBIERSKY, Jörg, D-46282 Dorsten (DE); FUHRMANN, Edgar, D-44579 Castrop-Rauxel (DE); BRÜGGEMANN, Wolfgang, D-44581 Castrop-Rauxel (DE)
(74) Vertreter: Cohausz & Florack
(86) Internationale Anmeldenummer: EP9902191
(87) Internationale Veröffentlichungsnummer: WO99050212

(56) Entgegenhaltungen:
- US-A- 4 365 099

## Beschreibung

Die Erfindung betrifft ein Verfahren zum Entfernen von Metallionen aus Kresol oder Kresol enthaltenden Aromatengemischen.

Kresolgemische sind Isomerengemische aus o-, m- und p-Kresol (Methylphenol). Sie fallen unter anderem als Produkt bei der Destillation von Steinkohlenteer an. Je nach Herstellungs- und Lagerungsverfahren können Kresolgemische mehr oder weniger große Mengen an Metallionen als Verunreinigungen enthalten. So enthält ein typisches, aus der Destillation von Steinkohlenteer gewonnenes Kresolgemisch in der Regel 30 bis 70 ppb (1 ppb = 1 mg/t) Natrium, 20 bis 40 ppb Eisen und 1 bis 10 ppb Chrom.

Problematisch sind derartige Verunreinigungen durch Metallionen beispielsweise bei der Verwendung von Kresol als Monomer zur Herstellung von Kresolharzen für die Mikrochipindustrie. Die für diesen Zweck benötigten Kresolgemische dürfen eine Konzentration höchstens 20 ppb pro Metallion aufweisen.

Die US 4 365 099 beschreibt die Entfernung von metallischen Verunreinigungen aus phenolischen Verbindungen wie Kresolen, die als Ausgangsmaterial zur Synthese von Bisphenolen eingesetzt werden sollen. Zur Entfernung der Metalle wird vorgeschlagen, diese mit einem mit Metallen Chelatkomplexe bildenden Harz zu behandeln. Diese Verfahrensweise eignet sich jedoch nicht zur Entfernung von Alkalimetallionen wie Natrium. Darüber hinaus sind diesem Dokument keine Angaben zur Anfangskonzentration der Metalle und zur Konzentration der Metalle nach der Behandlung zu entnehmen.

Folglich liegt der Erfindung die Aufgabe zugrunde, ein Verfahren zum Entfernen von Metallionen aus Kresolgemischen bereitzustellen, durch das die Metallionenkonzentrationen im Kresolgemisch auf < 20 ppb pro Metallion reduziert werden. Das Verfahren soll ferner möglichst einfach und kostengünstig durchführbar sein.

Gelöst wird diese Aufgabe dadurch, daß man in einem Kresolgemisch oder Kresol enthaltenden phenolischem Gemisch durch Zugabe von Wasser einen Wassergehalt von mindestens 0,5 Gew.% einstellt und das Kresol/Wassergemisch anschließend über einen sauren Ionenaustauscher leitet.

Insbesondere ist das erfindungsgemäße Verfahren zur Reinigung von Kresol oder Kresol enthaltenden Aromatengemischen geeignet, die eine Ausgangsmetallionenkonzentration von 0,01 bis 0,1 ppm (10 bis 100 ppb) pro Metallion aufweisen.

Neben der gewünschten Verringerung der Metallionenkonzentration besitzt das erfindungsgemäße Verfahren den weiteren Vorteil, daß die Konzentration von im Kresolgemisch gegebenenfalls enthaltenen Basen wie Pyridin, Anilin und Chinolin deutlich reduziert wird. Zur Verringerung der Verunreinigung durch Metallionen war auch die Destillation der Kresolgemische in Betracht gezogen worden. Die Destillation erwies sich jedoch als nachteilig, weil dadurch eine Verschiebung der mengenmäßigen Anteile der Bestandteile des Kresolgemischs hervorgerufen wurde. Ferner war die Reduzierung der Metallionenkonzentration deutlich weniger effektiv.

Es hat sich gezeigt, daß unbehandelte Kresolgemische den Ionenaustauscher nach kurzer Zeit für die Entfernung von Metallionen im ppb-Bereich unbrauchbar machen. Überraschend wurde festgestellt, daß dieses Problem nach Zugabe geringer Mengen Wasser zum Kresolgemisch nicht auftritt und der Ionenaustauscher über einen langen Zeitraum aktiv bleibt. Gemäß einer bevorzugten Ausführungsform der Erfindung wird der Wassergehalt im Kresolgemisch, insbesondere wenn dieses als Ausgangsmaterial für Polymere in der Mikrochipindustrie eingesetzt werden sollen, auf bis zu 15 Gew.%, insbesondere bis zu 10 Gew.% oder 1 bis 5 Gew.% Wasser, bezogen auf das Gewicht des Kresolgemischs oder Kresol enthaltenden Aromatengemischs, eingestellt.

Als Ionenaustauscher kommen alle Arten von sauren Ionenaustauschern in Frage. Erfindungsgemäß besonders bevorzugt sind stark saure Ionenaustauscher, Ionenaustauscherharze - auf Basis eines Styrol/Divinylbenzol-Copolymers. Die Verwendung eines stark sauren Ionenaustauscherharzes ist inbesondere im Hinblick auf die Beseitigung von schwer zu entfernenden Ionen wie Na⁺ vorteilhaft. Die Ionenaustauscherharze weisen vorzugsweise eine makroporöse Struktur und/oder einen hohen Vernetzungsgrad der Polymermatrix auf.

Ein Beispiel für einen erfindungsgemäß besonders bevorzugten Ionenaustauscher ist Amberlyst® 15 (Rohm & Haas Company). Amberlyst® 15 ist ein stark saurer, makroporöser Ionenaustauscher auf Basis eines Styrol/Divinylbenzol-Copolymers mit einem Divinylbenzolgehalt von 20 % und einer -SO₃H Konzentration (aktive Gruppen) von 4,7 eq/kg. Amberlyst® 15 weist eine Katalysatoroberfläche von 45 m²/g, einen Wassergehalt von 51-56 % und ein Schüttgewicht von 770 g/l auf.

Die sauren Ionenaustauscher werden, wie bei Reaktionen dieser Art üblich, vor Inbetriebnahme durch Behandlung mit Säuren wie Schwefelsäure aktiviert. Vor und nach der Aktivierung mit Säure wird der Ionenaustauscher in der Regel mit destilliertem Wasser gewaschen. Es hat sich als zweckmäßig erwiesen, den Ionenaustauscher nach dem Waschen mit destillierten Wasser nicht mit Methanol oder ähnlichen Lösungsmitteln zu trocknen.

Die Menge des Ionenaustauschers sowie die Durchflußgeschwindigkeit hängen grundsätzlich vom Verunreinigungsgrad des zu reinigenden Kresolgemischs ab. So ist bei einem hohen Verunreinigungsgrad des Kresolgemischs eine größere Menge des Ionenaustauschers und/oder eine niedrigere Durchflußgeschwindigkeit erforderlich als bei einem geringen Verunreinigungsgrad. Als praxisgerecht hat es sich erwiesen für 100 kg Ausgangsmaterial 1 kg Ionenaustauscher einzusetzen. Nach einer bestimmten Durchlaufmenge muß der Ionenaustauscher durch übliche Verfahren regeneriert werden.

Die Temperatur zur Durchführung des erfindungsgemäßen Verfahrens ist unkritisch. Hier kommt es in erster Linie darauf an, zusätzliche Energiekosten zu vermeiden, weshalb meist die Umgebungstemperatur gewählt werden wird.

Das Produkt des erfindungsgemäßen Verfahrens ist ein Kresolgemisch, das Phenol und weitere alkylierte Aromaten enthalten kann und insbesondere einen Metallionengehalt von weniger als 20 ppb pro Metallion aufweist. Die im Produktgemisch enthaltene Wassermenge entspricht der im Ausgangsmaterial vor Durchfluß durch den Ionenaustauscher eingestellten Wassermenge.

Nachfolgend wird die Erfindung anhand von Ausführungsbeispielen näher erläutert.

### Beispiel 1

Ein aus der Steinkohlenteerdestillation gewonnenes Kresolgemisch mit einer Na-Ionenkonzentration von 70 ppb und einer Fe-Ionenkonzentration von 21 ppb wurde mit 10 Gew.% destilliertem Wasser versetzt und bei einer Durchlaufgeschwindigkeit von 400 ml/h durch einen 38 x 180 mm großen Durchflußreaktor, der mit 150 g eines zuvor nacheinander mit 1 1 Wasser, 2 1 Schwefelsäure (12 %) und 1 1 Wasser behandelten Ionenaustauscher vom Typ Amberlyst® 15 H (Trockengewicht: 89,1 g) gefüllt war, geleitet. Bis zu einer Durchflußmenge von 10.000 ml wies das über den Ionenaustauscher geleitete Kresolgemisch Metallionengehalte von *c*(Na) 8 ppb und *c*(Fe) < 10 ppb auf. Nach einer Durchflußmenge von 20.000 ml lagen die Metallionengehalte bei *c*(Na) 16 ppb und *c*(Fe) < 10 ppb.

### Beispiel 2

Ein aus der Steinkohlenteerdestillation gewonnenes Kresolgemisch mit einer Na-Ionenkonzentration von 26 ppb, einer Fe-Ionenkonzentration von 22 ppb und einer Basenkonzentration von 85 ppm wurde wie in Beispiel 1, jedoch ohne vorherige Wasserzugabe, durch den Amberlyst® 15 Ionenaustauscher geleitet. Nach einer Durchflußmenge von etwa 2500 ml wies das über den Ionenaustauscher geleitete Kresolgemisch Gehalte von *c*(Na) 7 ppb, *c*(Fe) < 10 ppb und *c*(Base) 8 ppm auf. Nach einer Durchflußmenge von 11.000 ml wies das Kresolgemisch wieder deutlich höhere Metallionenkonzentration von *c*(Na) 11 ppb und c(Fe) 21 ppb auf. Dies deutet auf einen schnellen Verbrauch des Ionenaustauschers unter wasserfreien Bedingungen hin. Nach einer Durchflußmenge von 10.000 ml wurde mit *c*(Na) 19 ppb und *c*(Base) 12 ppm eine Erhöhung der Natrium- und Basenkonzentration beobachtet. Nach einer Durchflußmenge von 12.500 ml überschritt die Natriumkonzentration mit *c*(Na) 22 ppb die für die Verwendung in der Mikrochipherstellung zulässige Höchstgrenze, wobei die Fe- und Basenkonzentration nach dieser Durchflußmenge *c*(Fe) 12 ppb und *c*(Base) 12 ppm betrug. Dies deutet auf einen schnellen Verbrauch des Ionenaustauschers unter wasserfreien Bedingungen hin.

## Patentansprüche

1. Verfahren zum Entfernen von Metallionen aus Kresol oder Kresol enthaltenden Aromatengemischen, **dadurch gekennzeichnet, daß** man in dem Kresolgemisch durch Zugabe von Wasser zunächst einen Wassergehalt von mindestens 0,5 Gew.% einstellt und das Kresol/Wassergemisch anschließend über einen sauren Ionenaustauscher leitet.

2. Verfahren nach Anspruch 1, **dadurch gekennzeichnet, daß** der Wassergehalt des Kresolgemischs auf 1 bis 10 Gew.% eingestellt wird.

3. Verfahren nach Anspruch 1 oder 2, **dadurch gekennzeichnet, daß** der Ionenaustauscher stark sauer ist.

## Claims

1. A method for removing metal ions from cresol or from mixtures of aromatic compounds containing cresol, wherein the water content of the cresol mixture is adjusted to at least about 0.5 per cent by weight by the addition of water and the cresol-and-water mixture is thereafter passed over an acidic ion exchanger.

2. A method as claimed in claim 1, wherein the water content of the cresol mixture is adjusted to about 1 to 10 per cent by weight.

3. A method as claimed in claim 1 or 2, wherein the ion exchanger is strongly acidic.

## Revendications

1. Procédé d'élimination d'ions métaux de crésol ou de mélanges d'aromatiques contenant du crésol, **caractérisé en ce que** l'on ajuste d'abord une teneur en eau d'au moins 0,5% en poids, par addition d'eau au mélange de crésol et que le mélange crésol/eau est ensuite conduit sur un échangeur d'ions acide.

2. Procédé suivant la revendication 1, **caractérisé en ce que** la teneur en eau du mélange de crésol est ajustée de 1 à 10% en poids.

3. Procédé suivant la revendication 1 ou 2, **caractérisé en ce que** l'échangeur d'ions est un acide fort.
